# EUROPEAN PATENT APPLICATION

(11) **EP 4 847 629 A1**
(43) Date of publication of application: **02.12.2026**
(21) Application number: 25745265.6
(22) Date of filing: 24.01.2025
(51) Int. Cl.: C12Q 1/6869, C12Q 1/6827

(54) **METHOD FOR VALIDATION AND FALSE POSITIVE ERROR ANALYSIS OF NEXT-GENERATION SEQUENCING PANEL BY USING DNA MIXTURE**

(30) Priority: 26.01.2024 KR 20240012706
(71) Applicant: National Cancer Center, Goyang-si, Gyeonggi-do 10408 (KR)
(72) Inventor: HONG, Kyeong Man, Paju-si Gyeonggi-do 10895 (KR); KIM, Jong Kwang, Goyang-si Gyeonggi-do 10356 (KR); MOON, Youngbeen, Goyang-si Gyeonggi-do 10307 (KR)
(74) Representative: J A Kemp LLP
(86) International application number: PCT/KR2025/001552
(87) International publication number: WO 2025/159599

(57) **Abstract**

The present invention relates to a method for validation and false positive error analysis of a next-generation sequencing (NGS) panel by using mixed DNA, and to a composition comprising mixed DNA for validation of a next-generation sequencing (NGS) panel. The method for validation and false positive error analysis of a next-generation sequencing (NGS) panel by using mixed DNA according to the present invention has an effect of evaluating the appropriateness of an NGS analysis result by analyzing false positive errors from a result of analyzing variations, and thus such a method for verification and false positive error analysis of a next-generation sequencing (NGS) panel by using mixed DNA and a composition containing mixed DNA for validation of a next-generation sequencing (NGS) panel can be helpfully used for validation of the next-generation sequencing (NGS) panel.

## Description

### [Technical Field]

The present disclosure relates to a method for validation and false positive error analysis of a next-generation sequencing (NGS) panel using mixed DNA, and to a composition for validation of a next-generation sequencing (NGS) panel, including mixed DNA.

### [Background Art]

In order to develop an NGS panel and apply the same to clinical practice, it is necessary to evaluate whether the accuracy and sensitivity of the panel reach desired levels. To this end, the U.S. Food and Drug Administration (FDA) has published guidelines for validation of NGS panels, and the Center for Medicare & Medicaid Services (CMS) monitors panels, with the guidelines and monitoring methods being continuously updated. In addition, the College of American Pathologists (CAP) conducts certification through CAP surveys, and in Korea, the Ministry of Food and Drug Safety (MFDS) certifies NGS tests. However, these certification bodies do not provide tools capable of measuring the sensitivity and specificity of NGS panels and performing relative evaluations thereof.

There is a method introduced by Frampton et al. in Nature Biotechnology in 2013 for measuring the sensitivity and specificity of an NGS panel (hereinafter, the "Frampton method"), and the Frampton method has been predominantly used in subsequent studies to date (Shin et al., Nature Comm 2017; and Froyen et al., Cancers 2022). The Frampton method is a method for validating an NGS panel using a DNA pool in which DNAs from HapMap cell lines having well-known sequences are mixed, or a DNA pool in which DNAs from cancer cell lines are mixed. However, the Frampton method also has difficulty in evaluating the sensitivity of an NGS panel due to errors arising from differences between the expected allelic fractions and the measured values in NGS results obtained by accurately mixing about 10 DNAs in equal amounts during the dilution process. In particular, the Frampton method has a limitation in that it cannot measure specificity, and therefore has not been applied and used for establishing guidelines.

In addition to the Frampton method, methods have been introduced to measure the sensitivity of mutation detection by mixing multiple sequences containing mutations, diluting the mixture into normal genomic DNA at a certain ratio, and testing them with an NGS panel to determine the dilution ratio at which the mixed mutations can be detected. A method has been commercialized and introduced in which a sequence mixture containing 40 variants in 28 genes is provided (Seraseq^{®} Tumor Mutation DNA Mix v2 AF 10 HC, SeraCase, Mildford), and is mixed at a desired ratio with wild-type DNA (GM24385) from a person known not to have the variants present in the mixture and then used for NGS panel analysis, thereby enabling detection of mutations of 1% or less (Vicente-Garces et al., Frontiers in Mol Biosciences, 2022). In addition, the same company provides DNA mixtures in which wild-type DNA is mixed with each of 25 variants in 16 genes at different concentrations so that the sensitivities of the variants can be measured, thereby enabling evaluation of the sensitivity of an NGS panel by monitoring ctDNA (Seraseq^{®} ctDNA Complete^{™} Reference Material, SeraCase, Mildford). However, these standard samples have limitations in that they can be used only for testing specific NGS panels and cannot be applied to other NGS panels, and critically, they cannot measure specificity.

Under circumstances in which no standard material capable of universally measuring sensitivity and specificity is available, the present inventors conducted studies to determine the sensitivity and specificity of NGS using a DNA mixture standard sample in which homozygote DNA, heterozygote DNA, or control genomic DNA is mixed, and to improve analysis parameters for sensitivity and specificity, and in particular, developed a groundbreaking method capable of improving false positive and false negative errors in NGS, thereby completing the present disclosure.

### [Disclosure]

### [Technical Problem]

The present disclosure aims to solve the above-described problems and other problems associated therewith.

An exemplary object of the present disclosure is to provide a method for validation of a next-generation sequencing (NGS) panel through analysis of a limit of detection, the method comprising:
(a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting Ho-N pair alleles (Homozygote-Null pair alleles) and/or He-N pair alleles (Heterozygote-Null pair alleles) from the results of the next-generation sequencing (NGS) performed in the (a);
(c) analyzing a diluted variant detection rate, expressed as a percentage of the number of diluted variants found among the Ho-N pair alleles and He-N pair alleles in the DNA mixture sample, based on the selected Ho-N pair alleles and/or He-N pair alleles; and
(d) analyzing a limit of detection.

Another exemplary object of the present disclosure is to provide a method for validation of a next-generation sequencing (NGS) panel through analysis of a false positive error rate, the method comprising:
(a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting false positive variants from the results of the next-generation sequencing (NGS) performed in the (a); and
(c) analyzing a false positive error rate in the DNA mixture sample based on the selected false positive variants.

Still another exemplary object of the present disclosure is to provide a method for validation of a next-generation sequencing (NGS) panel, the method comprising:
(a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting Ho-N pair alleles (Homozygote-Null pair alleles) and/or He-N pair alleles (Heterozygote-Null pair alleles) from the results of the next-generation sequencing (NGS) performed in the (a);
(c) analyzing a diluted variant detection rate, expressed as a percentage of the number of diluted variants found among the Ho-N pair alleles and He-N pair alleles in the DNA mixture sample, based on the selected Ho-N pair alleles and/or He-N pair alleles; and
(d) comparing and analyzing an expected VAF and an actual VAF according to the dilution ratio.

Yet another exemplary object of the present disclosure is to provide a method for validation of a next-generation sequencing (NGS) panel, the method comprising:
(a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting Ho-N pair alleles (Homozygote-Null pair alleles) and/or He-N pair alleles (Heterozygote-Null pair alleles) from the results of the next-generation sequencing (NGS) performed in the (a);
(c) analyzing a diluted variant detection rate, expressed as a percentage of the number of diluted variants found among the Ho-N pair alleles and He-N pair alleles in the DNA mixture sample, based on the selected Ho-N pair alleles and/or He-N pair alleles; and
(d) analyzing deviations in variant read counts for respective chromosomal regions used for detection of diluted variants in (c) above.

Still another exemplary object of the present disclosure is to provide a composition for validation of a next-generation sequencing (NGS) panel, comprising a DNA mixture in which two or more cell-derived DNAs selected from the group consisting of homozygote DNA, control genomic DNA, and combinations thereof are mixed.

Still another exemplary object of the present disclosure is to provide a composition for validation of a next-generation sequencing (NGS) panel, comprising a mixture in which two or more cells selected from the group consisting of homozygote cells, control cells, and combinations thereof are mixed.

The technical problems to be achieved according to the technical spirit of the invention disclosed herein are not limited to the above-mentioned problems, and other problems not mentioned will be clearly understood by those skilled in the art from the following description.

### [Technical Solution]

The technical solution will be described in detail as follows. Meanwhile, descriptions and embodiments disclosed in the present application may also be applied to other descriptions and embodiments, respectively. That is, all combinations of the various elements disclosed in the present application fall within the scope of the present application. In addition, it cannot be said that the scope of the present application is limited by the specific descriptions described below.

In order to solve the problems described in the Background Art, the present inventors have conducted studies to determine the sensitivity and specificity of NGS using a DNA mixture standard sample in which homozygote DNA or control genomic DNA is mixed. During the course of the studies, the present inventors found that the information obtainable from the analysis results of sensitivity and specificity was limited, and thus further conducted studies to improve analysis parameters.

For example, when comparing the numbers of false positive variants according to respective NGS methods, it was difficult to directly compare relative specificities due to differences in the sizes of the respective target sequences. However, when calculating a false positive error rate by dividing the number of false positive errors by the number of bases in the target sequence of a particular NGS method, the specificities of multiple NGS methods could be readily compared. Furthermore, rather than separately determining a limit of detection for Ho-N pair alleles (Homozygote-Null pair alleles) and He-N pair alleles (Heterozygote-Null pair alleles), determining the limit of detection by integrating them is more efficient and allows for an intuitive assessment of errors.

In addition, during NGS analysis, various cases were found in which a false positive variant occurred in alleles other than an N-N (Null-Null) pair or a reference base-reference base pair (R-R pair). Specifically, a false positive may occur in alleles in which only a variant is found (variant-variant pair, V-V pair) or in alleles in which a reference base and a variant are mixed (reference base-variant mixed pair, R-V mixed pair or R-V pair). However, during the analysis process, it was found that the detection rate of a reference base as false positives in V-V pairs is significantly higher than that of other false positive variants. Since such errors occur due to bias in bioinformatic analysis, it was determined that excluding such errors and then obtaining the overall false positive variants would be advantageous for comparing and analyzing false positive errors across various NGSs.

In addition to parameters such as sensitivity and specificity, the present inventors newly found that, when analyzing the linear relationship between an expected variant allelic fraction (VAF) and a VAF measured by NGS, there were some cases where a linear relationship was not found and the line was bent, and thus such analysis would be helpful in measuring errors of NGS. In addition, when the read depths of variants for respective chromosomes are presented, it is possible to determine the distribution of capture or amplification efficiencies in an NGS kit for the respective chromosomes. From such results, if a deviation among chromosomal positions is large, it may be determined that a false negative error rate is high and improvement is required. In another analysis, when regions in which false positive errors occurred are presented according to chromosomal regions, there are alleles exhibiting particularly high false positive rates. Since variant results of such alleles are generated by errors, it is desirable to indicate an extent of the false positive error rate in an NGS result report so that the extent can be known for specific alleles. The present disclosure enables the finding of such false positive errors and newly found that alleles exhibiting high false positive rates may be used as parameters for improving the specificity of NGS.

In one aspect for achieving the above object, the present disclosure provides a method for validation of a next-generation sequencing (NGS) panel through analysis of a limit of detection, the method comprising: (a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting Ho-N pair alleles (Homozygote-Null pair alleles) and/or He-N pair alleles (Heterozygote-Null pair alleles) from the results of the next-generation sequencing (NGS) performed in the (a);
(c) analyzing a diluted variant detection rate, expressed as a percentage of the number of diluted variants found among the Ho-N pair alleles and He-N pair alleles in the DNA mixture sample, based on the selected Ho-N pair alleles and/or He-N pair alleles; and
(d) analyzing a limit of detection.

As used herein, the term "allele" refers to a gene that forms a pair of homologous chromosomes and has different traits, and means a DNA sequence of an allele. Each allele exists in a homozygote state and a heterozygote state, wherein the "homozygote" allele refers to a case where two genes in an individual have alleles with the same base, and a "heterozygote" allele refers to a case where an individual or cell has alleles with different bases.

As used herein, the term "homozygote cell" refers to a cell in which almost all alleles are composed of homozygote alleles except for a few alleles with exceptions, and the term "homozygote DNA" refers to DNA isolated from a homozygote cell.

In addition, the term "heterozygote cell" refers to a cell in which traits inherited from each parent are different so that many alleles are composed of heterozygote alleles, and the term "heterozygote DNA" refers to DNA isolated from a heterozygote cell, and is a concept distinguished from a heterozygote trait.

In an embodiment of the present disclosure, the DNA mixture sample in which two or more cell-derived DNAs selected from the group consisting of homozygote DNA, control genomic DNA, and combinations thereof are mixed may be a mixture of two or more homozygote DNAs having different variants; a mixture of homozygote DNA and control genomic DNA; or a combination thereof.

An example of the two or more homozygote DNAs having different variants may include respective homozygote DNAs isolated from H. mole cells derived from two different patients, wherein the respective homozygote DNAs may exhibit different variants, insertions, and deletions (indels) constituting them.

In an embodiment of the present disclosure, the homozygote DNA may be DNA isolated from a hydatidiform mole (H. mole) cell line, a parthenogenetic cell line, or a cell in which most alleles are homozygote alleles.

In an embodiment of the present disclosure, the "control genomic DNA" may be homozygote DNA; heterozygote DNA; or genomic DNA isolated from blood cells or tissue cells of a normal subject, cultured blood cells, tissue cells or cancer cells, or blood cells or cancer tissue cells of a patient. In addition, the control genomic DNA may be circulating tumor DNA (ctDNA). In addition, the control genomic DNA may be synthetic DNA or cloned DNA.

In an embodiment of the present disclosure, a dilution ratio of the homozygote DNA and the control genomic DNA may be selected from the group consisting of 1:99 to 99:1. Preferably, the dilution ratio of the homozygote DNA and the control genomic DNA may be selected from the group consisting of 1:9999, 2.5:9997.5, 5:9995, 1:999, 2.5:997.5, 5:995, 1:99, 2.5:97.5, 5:95, 10:90, 20:80, 50:50, 80:20, 90:10, 95:5, 97.5:2.5, 99:1, 995:5, 997.5:2.5, 999:1, 9995:5, 9997.5:2.5, and 9999:1, but is not limited thereto.

In an embodiment of the present disclosure, the homozygote DNA or the control genomic DNA may be isolated from fresh cells, fresh tissues, or formalin fixed paraffin embedded (FFPE) cellular tissues.

As used herein, the term "fresh cell" refers to a living cell obtained from culture and may also include a frozen cell that has not been subjected to chemical treatment, and the term "fresh tissue" refers to a tissue directly isolated from a living body and may also include a frozen tissue that has not been subjected to chemical treatment.

The "formalin fixed paraffin embedded (FFPE) cellular tissue" in the present disclosure may be obtained without limitation to methods known in the art, and may be obtained, for example, through tissue fixation, embedding processes, and the like.

As used herein, the term "next-generation sequencing (NGS)" refers to a technology capable of analyzing millions of sequences simultaneously, and is also referred to as massively parallel sequencing or high-throughput sequencing. In the present disclosure, the terms "next-generation sequencing" and "NGS" are used interchangeably.

As used herein, the term "Ho-N pair allele (Homozygote-Null pair allele)" refers to a pair in which, among selected alleles, the alleles of homozygote DNA (or homozygote cells) including H. mole DNA and control genomic DNA (or control genomic cells) are respectively composed of a homozygous variant and a null having no variant, the term "He-N pair allele (Heterozygote-Null pair allele)" refers to a pair in which, among selected alleles, the homozygote DNA is null and the control genomic DNA is composed of a heterozygote variant.

More specifically, a criterion for selecting a He-N pair allele may vary depending on the types and number of DNAs included in the DNA mixture constituting a sample. That is, in a sample in which heterozygote DNA and homozygote DNA are mixed at a ratio of 50:50, when the homozygote DNA has a reference base (or a null allele) and the heterozygote DNA has a heterozygote variant, it is selected as a He-N pair, in this case, a ratio of the reference base to the variant allele is 3:1, but when a plurality of mixed samples are mixed, an allele ratio of the reference base to the variant may not be 3:1 even when the respective single DNAs are mixed in equal amounts. For example, when a reference standard material is prepared by mixing DNAs of two homozygote cells and one type of control genomic DNA in equal amounts in a DNA mixture, if a reference base appears in both homozygote DNAs at a specific allele and a heterozygote variant in which a reference base and a variant are mixed appears in the control genomic DNA, a ratio of the null allele to the variant allele becomes 5:1.

As used herein, the term "informative allele" refers to alleles having predetermined conditions for analyzing sensitivity or specificity, for example, Ho-N pair alleles and He-N pair alleles can be said to be informative alleles used for measuring sensitivity. In such pairs, a variant base or a reference base is present only in one of homozygote DNA (DNA1) and control genomic DNA (DNA2), and sensitivity may be measured by determining whether a diluted base among the informative alleles is detected in the analysis result of a mixed DNA sample. In addition, informative alleles required for measuring specificity may be classified into an R-R pair, a V-V pair, a V-R mixed pair, and the like, and specificity may be measured by detecting, as a false positive allele, a base that is not present in DNA1 and DNA2 but appears in the analysis result of the mixed DNA sample. Such informative alleles vary depending on the state of the alleles constituting the homozygote cells and the control genomic cells, such as variant, null, or heterozygote, when a reference standard material is prepared by mixing homozygote cells and control genomic cells.

As used herein, the term "diluted variant detection rate" is defined as indicating, as a percentage, the number of diluted variants found at an expected dilution ratio of variants among Ho-N pair alleles or He-N pair alleles in a DNA mixture sample in which homozygote DNA and control genomic DNA are mixed.

As used herein, the term "limit of detection" refers to a value of the minimum variant allelic fraction (VAF) at which the detection rate is 90% or more, and in general, the detection rate serving as a criterion for the limit of detection may be lowered or increased. Here, for the condition that the detection rate defining the limit of detection is 90% or more, various values such as 95% or 99% may be used depending on conditions.

As used herein, the term "VAF (variant allelic fraction)" refers to a value obtained by dividing the read count of sequences detected as variants at a specific allele site by the total read count obtained by adding the sequences detected as variants and reference sequence bases at the specific allele site.

As another aspect for achieving the above object, the present disclosure provides a method for validation of a next-generation sequencing (NGS) panel through analysis of a false positive error rate, the method comprising:
(a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting false positive variants from the results of the next-generation sequencing (NGS) performed in the (a); and
(c) analyzing a false positive error rate in the DNA mixture sample based on the selected false positive variants.

The "homozygote DNA," "control genomic DNA," and "next-generation sequencing (NGS)" are as described above.

In an embodiment of the present disclosure, the DNA mixture sample in which two or more cell-derived DNAs selected from the group consisting of homozygote DNA, control genomic DNA, and combinations thereof are mixed may be a mixture of two or more homozygote DNAs having different variants; a mixture of homozygote DNA and control genomic DNA; or a combination thereof.

As used herein, the term "false positive variant" refers to a variant in which a test result that should originally be negative is erroneously determined to be positive, and is defined as a case in which a base not detected in other analyses of a homozygote DNA sample or a control genomic DNA sample is detected in the NGS analysis result of the sample.

In an embodiment of the present disclosure, the false positive variant may be a case in which a variant is shown to exist in the result of analyzing a DNA mixture sample by NGS among null alleles in which no variant is found in any of two or more cell-derived DNAs selected from the group consisting of homozygote DNA, control genomic DNA, or combinations thereof.

In an embodiment of the present disclosure, the false positive variant may be a case in which a variant other than the V-V pair or a reference base is found in the result of analyzing a DNA mixture sample by NGS among alleles (V-V pair) in which no reference base is found and only variants are all found in two or more cell-derived DNAs selected from the group consisting of homozygote DNA, control genomic DNA, or combinations thereof.

In an embodiment of the present disclosure, the false positive variant may be a case in which a new variant other than the bases constituting an R-V pair is found in the result of analyzing a DNA mixture sample by NGS among alleles (R-V pair alleles) in which a reference base and a variant are mixed in two or more cell-derived DNAs selected from the group consisting of homozygote DNA, control genomic DNA, or combinations thereof.

In the present disclosure, the (c) may further comprise removing a VVR false positive error result in which a reference base is found in a V-V pair. The "VVR false positive error" refers to a false positive error generated by calling as a reference base in a V-V pair.

In the present disclosure, step (c) may further comprise excluding error results of alleles in which false positive errors appear at a high frequency. The alleles in which false positive errors appear at a high frequency refer to alleles in which false positive errors intensively occur, such false positive errors arise due to errors generated in an NGS raw data production process, such as a difference in experimental materials used for producing raw data of NGS, a subtle difference in sample concentration in an experimental process using the materials, or a difference in PCR instruments, and such errors are unrelated to the performance of the NGS panel, the error results of alleles in which the false positive errors appear at a high frequency may be excluded in order to improve the accuracy of the NGS panel validation process.

In the present disclosure, step (c) may further comprise examining a batch effect.

As used herein, the term "batch effect" refers to a change in data caused by conditions other than biological factors that may occur during an experiment, in particular, when NGS samples are sent to the same company at different times and the results are analyzed, the term refers to a form of error in which the results are found differently depending on the time at which the samples were sent, while exhibiting a pattern different from differences found among samples sent at the same time. A specific example of a batch effect examination includes a case of reviewing whether a reference standard material is sent simultaneously with a clinical sample or a research sample to produce NGS raw data.

As another aspect for achieving the above object, the present disclosure provides a method for validation of a next-generation sequencing (NGS) panel, the method comprising:
(a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting Ho-N pair alleles (Homozygote-Null pair alleles) and/or He-N pair alleles (Heterozygote-Null pair alleles) from the results of the next-generation sequencing (NGS) performed in the (a);
(c) analyzing a diluted variant detection rate, expressed as a percentage of the number of diluted variants found among the Ho-N pair alleles and He-N pair alleles in the DNA mixture sample, based on the selected Ho-N pair alleles and/or He-N pair alleles; and
(d) comparing and analyzing an expected VAF and an actual VAF according to the dilution ratio.

The "homozygote DNA," "control genomic DNA," "next-generation sequencing (NGS)," "Ho-N pair allele," "He-N pair allele," "diluted variant detection rate," and "VAF (variant allelic fraction)" are as described above.

In an embodiment of the present disclosure, the DNA mixture sample in which two or more cell-derived DNAs selected from the group consisting of homozygote DNA, control genomic DNA, and combinations thereof are mixed may be a mixture of two or more homozygote DNAs having different variants; a mixture of homozygote DNA and control genomic DNA; or a combination thereof.

As another aspect for achieving the above object, the present disclosure provides a method for validation of a next-generation sequencing (NGS) panel, the method comprising:
(a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting Ho-N pair alleles (Homozygote-Null pair alleles) and/or He-N pair alleles (Heterozygote-Null pair alleles) from the results of the next-generation sequencing (NGS) performed in the (a);
(c) analyzing a diluted variant detection rate, expressed as a percentage of the number of diluted variants found among the Ho-N pair alleles and He-N pair alleles in the DNA mixture sample, based on the selected Ho-N pair alleles and/or He-N pair alleles; and
(d) analyzing deviations in variant read counts for respective chromosomal regions used for detection of diluted variants in (c) above.

The "homozygote DNA," "control genomic DNA," "next-generation sequencing (NGS)," "Ho-N pair allele," "He-N pair allele," and "diluted variant detection rate" are as described above.

In an embodiment of the present disclosure, the DNA mixture sample in which two or more cell-derived DNAs selected from the group consisting of homozygote DNA, control genomic DNA, and combinations thereof are mixed may be a mixture of two or more homozygote DNAs having different variants; a mixture of homozygote DNA and control genomic DNA; or a combination thereof.

As used herein, the term "variant read count" refers to the number of times each variant is read for a chromosomal region. When a deviation in read counts exist among chromosomal regions, the sensitivity of the NGS panel is relatively low and the reliability of the NGS results is reduced, and therefore, in order to validate an appropriate next-generation sequencing (NGS) panel, it is necessary to analyze deviations in variant read counts for chromosomal regions used for diluted variant detection.

As another aspect for achieving the above object, the present disclosure provides a composition for validation of a next-generation sequencing (NGS) panel, comprising a DNA mixture in which two or more cell-derived DNAs selected from the group consisting of homozygote DNA, control genomic DNA, and combinations thereof are mixed.

The "next-generation sequencing (NGS)" is as described above.

In an embodiment of the present disclosure, the DNA mixture in which two or more cell-derived DNAs selected from the group consisting of homozygote DNA, control genomic DNA, and combinations thereof are mixed may be a mixture of two or more homozygote DNAs having different variants; a mixture of homozygote DNA and control genomic DNA; or a combination thereof.

In an embodiment of the present disclosure, the homozygote DNA may be DNA isolated from a hydatidiform mole cell line or a parthenogenetic cell line.

In an embodiment of the present disclosure, the "control genomic DNA" may be homozygote DNA; heterozygote DNA; or genomic DNA isolated from blood cells or tissue cells of a normal subject, cultured blood cells, tissue cells or cancer cells, or blood cells or cancer tissue cells of a patient.

In an embodiment of the present disclosure, the DNA mixture may be isolated from fresh cells, fresh tissues, or formalin fixed paraffin embedded (FFPE) cellular tissues.

In an embodiment of the present disclosure, the composition may be a reference standard material for validation of a next-generation sequencing panel.

As another aspect for achieving the above object, the present disclosure provides a composition for validation of a next-generation sequencing (NGS) panel, comprising a mixture in which two or more cells selected from the group consisting of homozygote cells, control cells, and combinations thereof are mixed.

The "homozygote" and "next-generation sequencing (NGS)" are as described above.

In an embodiment of the present disclosure, the mixture in which two or more cells selected from homozygote cells, control cells, or combinations thereof are mixed may be a mixture of two or more homozygote cells having different variants; a mixture of two or more homozygote cells having different variants and control cells; a mixture of homozygote cells and control cells; or a combination thereof.

In an embodiment of the present disclosure, the homozygote cells or the control cells may be fresh cells or formalin fixed paraffin embedded (FFPE) cells.

In an embodiment of the present disclosure, the composition may comprise, in the form of a formalin fixed paraffin embedded (FFPE) cell block, a mixture in which two or more cells selected from homozygote cells, control cells, or combinations thereof are mixed.

The "formalin fixed paraffin embedded cell block" in the present disclosure may be obtained through a process of collecting, fixing, and embedding single cells or complex cells. In general, when formalin-fixed paraffin-embedded (FFPE) cellular tissues are used, differences may arise from the sensitivity and specificity results of T-NGS using a reference standard material prepared with DNA isolated from fresh cells or fresh tissues due to DNA fragmentation, and accordingly, in order to eliminate or reduce the occurrence of such differences in sensitivity and specificity, formalin-fixed paraffin-embedded (FFPE) cells may be prepared in the form of a cell block.

In an embodiment of the present disclosure, the homozygote cell may be a hydatidiform mole cell or a parthenogenetic cell.

In an embodiment of the present disclosure, the control cell may be a homozygote cell; a heterozygote cell; a blood cell or tissue cell of a normal subject; a cultured blood cell, tissue cell, or cancer cell; or a blood cell or cancer tissue cell of a patient.The blood cell or tissue cell of a normal subject; the cultured blood cell, tissue cell, or cancer cell; or the blood cell or cancer tissue cell of a patient may be a cell in which alleles are mixedly composed of heterozygotes and homozygotes.

In an embodiment of the present disclosure, the composition may be a reference standard material for validation of a next-generation sequencing panel.

### [Advantageous Effects]

The method for validation and false positive error analysis of a next-generation sequencing (NGS) panel using mixed DNA according to the present disclosure has an effect of evaluating the appropriateness of an NGS analysis result by analyzing false positive errors from a result of analyzing variants, and thus such a method for validation and false positive error analysis of a next-generation sequencing (NGS) panel by using mixed DNA and a composition containing mixed DNA for validation of a next-generation sequencing (NGS) panel can be usefully used for validation of the next-generation sequencing (NGS) panel.

### [Brief Description of Drawings]

FIG. 1 shows a variant detection rate at a theoretical variant allele fraction (VAF) of a Ho-He-N pair allele (X-axis: theoretical VAF (%) of a Ho-He-N pair allele; Y-axis: variant detection rate at which a variant is detected among Ho-He-N pair alleles having theoretical VAF values).
FIG. 2 shows types determined as false positive errors (DNA1 and DNA2 are homozygote DNA and control genomic DNA, respectively, and alphabets indicated in lowercase red letters represent bases not found in DNA1 and DNA2).
FIG. 3 shows false positive error rates analyzed according to analysis methods by company and NGS (X-axis: analysis methods according to company and NGS; Y-axis: false positive error rate (number of false positive errors/number of target bases)). A. False positive error rate in an R-R pair. B. False positive error rate in an R-V pair. C. False positive error rate in a V-V pair. D. False positive error rate in a V-V pair after removing a VVR error.
FIG. 4 compares differences in overall false positive error rates between results excluding VVR errors (VVR-) and results including VVR errors (VVR+) in which a reference base appears in a V-V pair in the in-house analysis results of company BB.
FIG. 5 shows sensitivity measurement results based on dilution ratios of a reference base (X-axis: allele fraction percentage (AF percentage) of a reference base; Y-axis: reference base detection rate).
FIG. 6 shows overall false positive error rates analyzed after removing reference base detection errors in a V-V pair (X-axis: analysis methods according to company and NGS; Y-axis: overall false positive error rate).
FIG. 7 shows correlations between expected values and measured values of dilution ratios of variant alleles in Ho-N pair alleles and He-N pair alleles according to each company and analysis method (X-axis: measured VAF values (actually observed VAFs) of variant alleles; Y-axis: expected VAF values according to dilution ratios).
FIG. 8 shows read count analysis results of variants analyzed from results of multiple NGS service providers (AA to DD) (X-axis: chromosome position numbers; Y-axis: read count; the median value is indicated at the center of the box, and the upper and lower portions of the box represent the 25% and 75% values of the read counts, respectively).
FIG. 9 shows the distribution of alleles in which false positive errors appear in the NGS results of company CC (X-axis: alleles in which false positive errors come out are listed in order according to chromosome regions; Y-axis: allele fractions of erroneous bases in an allele in which an error appears).
FIG. 10 shows a method for providing a reference standard material by mixing two different cells and preparing a formalin fixed paraffin embedded (FFPE) tissue block.

### [Modes for Carrying Out the Invention]

Hereinafter, the present disclosure will be described in more detail through the following examples. However, these examples are intended to illustrate the present disclosure, and the scope of the present disclosure is not limited thereto.

### Example 1. Sensitivity Analysis by Integrating Variant Alleles in Ho-N Pair and He-N Pair

### 1.1. Preparation of DNA Mixture

In the present disclosure, for validation of an NGS panel, sensitivity and specificity of NGS panels from three companies were compared and analyzed using, as reference standard materials, H. mole DNA (hydatidiform mole, DNA1) derived from a human hydatidiform mole, which is homozygote DNA, control genomic DNA (DNA2), which is heterozygote DNA derived from a patient's blood cells, and a sample of a mixture of these two DNAs. In order to use the isolated DNAs as a DNA mixture, multiple mixture complex samples were prepared by mixing H. mole DNA (DNA1) and control genomic DNA (DNA2) at various ratios. The H. mole DNA derived from a human hydatidiform mole used in the present disclosure was purchased from Coriell (NA07489, Camden, NJ), and the control genomic DNA was used by extracting genomic DNA from the blood of a collaborator with approval from the Institutional Review Board of the National Cancer Center.

### 1.2. NGS Analysis

The prepared H. mole DNA (DNA1), control genomic DNA (DNA2), and DNA mixtures in which H. mole DNA and control genomic DNA were mixed were sent to company AA, company BB, and company CC, respectively, and experiments were performed using the Novaseq 6000 NGS instrument of Illumina and the NGS panel of each company. Thereafter, a file in which variants were analyzed by a method prepared in each company was received from a FASTQ file obtained in the experiment, and validation of the NGS panel was performed.

### 1.3. Sensitivity Analysis of NGS Panels Through Analysis of Limit of Detection

In order to measure a limit of detection of a variant of NGS for each company by using a DNA mixture in which H. mole DNA and control genomic DNA are mixed as a reference standard material, first, all alleles corresponding to a Ho-N pair or a He-N pair were selected and defined as a Ho-He-N pair allele. In order to integrate the limit of detection result of each of the selected Ho-He-N pairs, a theoretical VAF (variant allele fraction) value of the He-N pair was calculated as half of that of Ho-N, so that results of each of the Ho-N pair or the He-N pair could be integrated into one result to calculate sensitivity.

Specifically, among the Ho-He-N pair alleles, in a case of a Ho-N pair, a theoretical VAF (or a VAF expected value) occupied by a variant among total alleles in a case of CH50 (a sample in which H. mole and control DNA are mixed at 50:50) becomes 50%, and in a case of a He-N pair, a VAF expected value of the CH50 sample becomes 25%. In this manner, all VAF expected values possessed by all Ho-He-N pair alleles even in samples of other mixing ratios were obtained, and a value obtained by dividing the number of variants actually detected at a specific VAF expected value among the Ho-He-N pair alleles by the number of Ho-He-N pair alleles having the specific VAF expected value was defined as a variant detection rate at the specific VAF expected value, and was calculated.

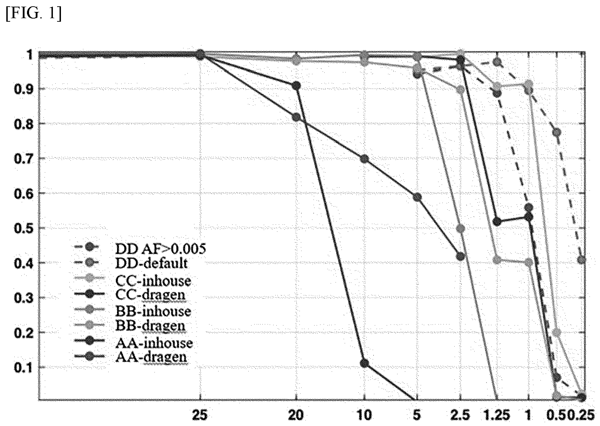

These variant detection rates were plotted as a graph, and a minimum VAF value at which a variant detection rate is 90% or more at each VAF expected value was determined as the limit of detection. FIG. 1 shows a variant detection rate at a theoretical VAF value for Ho-He-N pair alleles, wherein AA to DD indicate T-NGS kits of different companies, inhouse indicates that variant call was analyzed by an in-house bioinformatics analysis method used in each company, and dragen indicates that analysis was performed by using a DRAGEN system of Illumina company using a fastq file provided by each company in order to relatively compare results of bioinformatics methods. For example, AA-dragen indicates that a variant was analyzed by a DRAGEN system from T-NGS raw data obtained in company AA, and AA-inhouse indicates that a variant was analyzed by a bioinformatics analysis method used in company AA from T-NGS raw data obtained in company AA. In addition, DD-default indicates a variant call result analyzed by a bioinformatics analysis method used in company DD from T-NGS raw data obtained in company DD, and DD AF>0.005 indicates that a variant was analyzed by a bioinformatics analysis method used in company DD from T-NGS raw data obtained in company DD and only variants with AF>0.005 were selected. The X-axis indicates a theoretical VAF of a Ho-He-N pair allele, and the Y-axis indicates a variant detection rate at which a variant is detected among Ho-He-N pair alleles having theoretical VAF values. Estimating the limit of detection means that in a case of AA-inhouse, the limit of detection of VAF becomes 20%, and in a case of DD-default, it is about 1%. These results mean that a result of analyzing an company AA kit in its own company can detect about 90% when a variant occupies 20%, so that detection sensitivity of a variant can be said to be relatively low, and a result of company AA's own analysis using a company DD kit means that even a variant occupying 1% can be detected at about 90%, so that sensitivity can be said to be relatively high.

### Example 2. Method of Comparing False Positive Degree by Calculating False Positive Error Rate

When analyzing the number of false positive errors (FP errors) by analyzing variants in a mixture of homozygote DNA (DNA1) and control genomic DNA (DNA2), or a mixture of homozygote DNAs having different variant, the degree of false positives cannot be directly compared with each other when the number of targets used in NGS of several companies and the number of target bases to be analyzed are different. In order to solve this, false positive error rates (FP error rates) were calculated and compared in NGS results of different companies. The false positive error rate is a value obtained by dividing the number of false positives found in a DNA mixture sample by the number of bases of an NGS target gene, and even when the number of bases targeted by NGS is actually different among companies, a degree of a relative false positive error can be directly compared by easily using a false positive error rate generated from an NGS result. For reference, when the number of target genes is the same as in whole exome sequencing, a relative error degree may also be compared by the number of false positive errors.

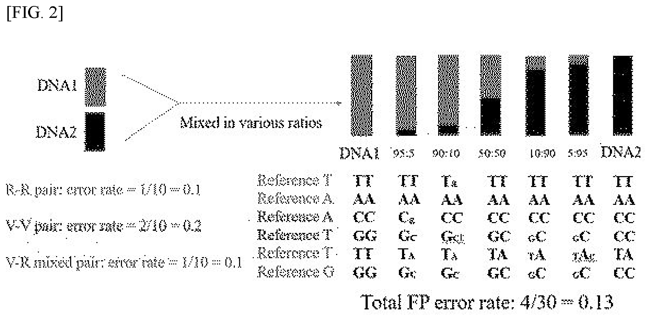

A false positive error was defined as a base that is not detected in analysis results of DNA1 and DNA2 but is newly detected in an analysis result of a mixture sample of DNA1 and DNA2.Depending on whether bases of DNA1 and DNA2 are a reference base or a variant base, a state of an allele may be classified into three pairs, which may occur in three types of pairs: 1) an R-R pair, a case where only a reference base is analyzed in DNA1 and DNA2; 2) a V-V pair, a case where only a variant is analyzed in both DNA1 and DNA2; and 3) a V-R mixed, a case where both a reference base and a variant base(s) are analyzed in DNA1 and DNA2.Since there is a difference in an appearance frequency and an error rate of false positive errors among these three types of pairs, the respective errors were measured separately (FIG. 2).

In order to obtain a false positive error rate using samples in which DNA1 and DNA2 are mixed, false positives were determined in each of the following three cases: 1) when a variant is found in an NGS analysis result of a specific DNA mixture sample among R-R pairs, it is determined that a pair allele has a false positive in the specific DNA mixture (when an error variant such as a appears, which is not a reference base in the R-R pair of FIG. 2). 2) When another variant or a reference base not found in DNA1 and DNA2 is found in an NGS analysis result of a specific DNA mixture sample among V-V pairs, it is determined that a V-V pair allele has a false positive in the specific DNA mixture (when an error variant or an error reference base such as g or t appears in the V-V pair of FIG. 2), and 3) when a new variant not found in DNA1 and DNA2 is found in an NGS analysis result of a specific DNA mixture sample among R-V mixed pairs, it is determined that an R-V pair mixed allele has a false positive in the specific DNA mixture (when an error variant such as c appears in the R-V mixed pair of FIG. 2).

Here, a reference base refers to a base corresponding to a specific chromosome position of a reference sequence used to compare DNA sequences of raw data analyzed in NGS. Various reference sequences may be used, but here, it especially refers to a reference sequence used for determining a chromosomal position and performing variant call during bioinformatics analysis in raw data to determine sensitivity and specificity. Examples of such a reference sequence include GRCh37 Genome Reference Consortium Human Build 37 (GRCh37) or hg19.

In order to measure a false positive error rate for the present disclosure, in a case of company BB, errors coming out from all sequences in a BED file were detected. In a case of the remaining companies, that is, company AA, company CC, and company DD, only errors found in an exon region of a target gene were detected, and even in in-house analysis results analyzed by each company itself, only errors in the exon region were selected to calculate a false positive error rate.

In order to obtain an R-R pair for measuring a false positive error rate, when a variant was not detected at all in H. mole DNA and control genomic DNA, it was included in the R-R pair. In a case of a V-V pair, all cases in which a variant was included, even to a slight extent, in two types of DNA such as DNA1 and DNA2 were included in the V-V pair. In a case of an R-V mixed pair, all cases in which both a reference base and a variant were included in two types of DNA such as DNA1 and DNA2 were included.

In order to calculate a false positive error rate, the number of alleles corresponding to each of a V-V pair and a V-R pair was calculated, and the number of false positive errors occurring in each pair was divided by the calculated number of alleles corresponding to each of the V-V pair and the V-R pair to calculate error rates in the V-V pair and the R-V pair, respectively. In order to calculate a false positive error rate of an R-R pair, in a case of company BB, the number obtained by subtracting the number of alleles of the V-V pair and the R-V pair from the number of bases of total sequences shown in a BED file was calculated as the number of total alleles of the R-R pair, and in a case of the remaining companies, the number of total alleles of the R-R pair was calculated in the same manner considering only the number of bases of exons of target genes. A value obtained by dividing the number of false positive errors coming out from each R-R pair region by the number of total alleles of the R-R pair was calculated as a false positive error rate of the R-R pair.

As an example of calculating a false positive error rate, in the result of FIG. 2, the number of R-R pairs is 10, and 1 false positive error occurred in a mixture sample, so that a false positive error rate of the R-R pair becomes 0.1 or 10%. Calculating in the same manner, an error rate of the V-V pair is 0.2, and in a case of the V-R mixed pair, an error rate is 0.1. In addition, the number of total alleles is 30 in total, and 4 errors in total were found in the mixture sample, so that a false positive error rate becomes 4/30 or 13.3%.

### Example 3. Method of Analyzing False Positive Error Rate after Removing False Positive Error Result in Which Reference Base Appears in V-V Pair

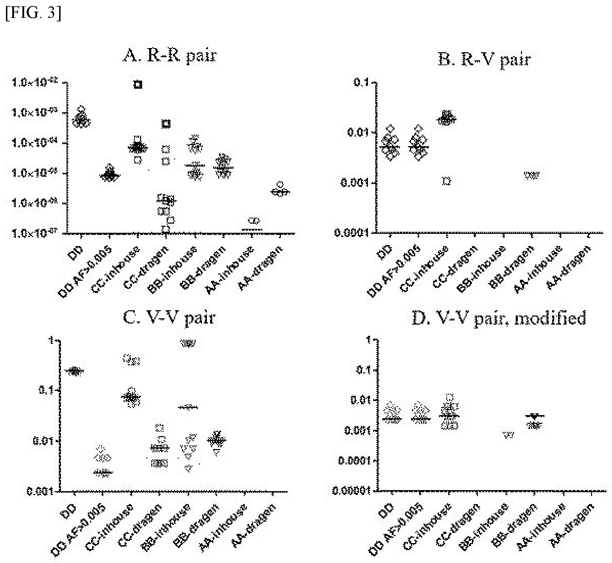

Actually, false positive error rates found in three pairs by analyzing NGS results of several companies were as shown in A to C of FIG. 3, wherein A is a false positive error rate in an R-R pair, B is a false positive error rate in an R-V pair, C is a false positive error rate in a V-V pair, and D is a final version of a false positive error rate analysis result after performing a conversion for excluding a VVR error in which a false positive comes out as a reference base in a V-V pair.

It was confirmed that an error rate of a result analyzed in the V-V pair is abnormally higher than that of analysis results in other pairs. This result may come out because errors of a PCR process are high in bases in which occurrence of errors is high. However, looking at BB-inhouse sample analysis data in detail, it was observed that false positive errors occur in most V-V pairs (an error rate is close to 1), and based on these results, it was inferred that this would be a systematic error occurring in a bioinformatics analysis process rather than an error occurring in an NGS experimental process of a specific company.

As a result of further analyzing a reason why false positive errors especially occur a lot in the V-V pair, there were especially many cases where appearance of a reference base in the V-V pair is determined as an error, and it can be seen that when such an error is removed, a false positive error rate is significantly reduced as shown in FIG. 3D. Such a case where a reference base appears in this way can be thought to be because a criterion for determining a specific allele as a variant and a criterion for determining the same as a reference base in a bioinformatics analysis process are different in an NGS result in which a variant and a reference base are mixed.

Explaining in more detail, when a reference base is a majority and a variant base is mixed only a little at a specific allele site of raw data, a condition for calling that there is a variant base at this allele site is strict; whereas when a majority of bases is a variant base and a minority of a reference base is mixed, a condition for calling that a reference base is mixed at a specific allele site is more permissive. As a result, when a reference base and a variant base are present at the same ratio in a specific V-V pair of raw data, the reference base is called much more than the variant base, and thus, false positive errors occurring as the reference base is called in the V-V pair sharply increase. Therefore, a false positive error occurring by calling as a reference base in a V-V pair in this way was defined as a VVR error.

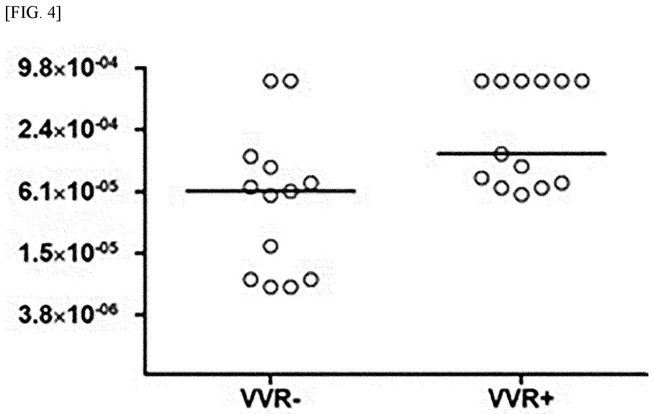

In order to examine how much such a VVR error affects a total false positive error rate, a total false positive error rate calculated by removing a VVR error and a total false positive error rate calculated without removing the same were evaluated, and as a result, a median value of a false positive error rate had a difference of two times or more depending on presence or absence of a VVR error (FIG. 4). This means that if a VVR error is not an error of an actual NGS result but an error of a bioinformatics analysis process performing a variant call, by removing VVR errors in which a reference base appears in a V-V pair, a total false positive error rate can be determined more accurately, and error rates of several different NGS panels can be compared and analyzed more accurately.

It was further verified whether a reference base bias phenomenon, in which a criterion for calling as true becomes different when a reference base appears and when a variant base appears in a specific sequence as described above, is found even in another analysis using data analyzed in the present study. Sensitivity may be measured from data of NGS, based on 1) a detection rate of a variant, and 2) a detection rate of a reference base, when there is a difference in sensitivity values obtained by these two methods, it means that such a reference base bias repeatedly appears, and this can be determined as an error occurring during a bioinformatics analysis process. In order to confirm a variant detection rate in such an analysis process, a detection rate of a diluted variant was calculated at an expected dilution ratio value of a variant. In contrast, in order to confirm a detection rate of a reference base, an expected reference base dilution ratio value representing the extent to which the reference base was diluted in alleles of each mixture sample was calculated, and the detection ratio of the reference base in alleles corresponding to the expected reference base dilution ratio value of a DNA mixture sample was calculated, defined as a reference base detection rate, and analyzed.

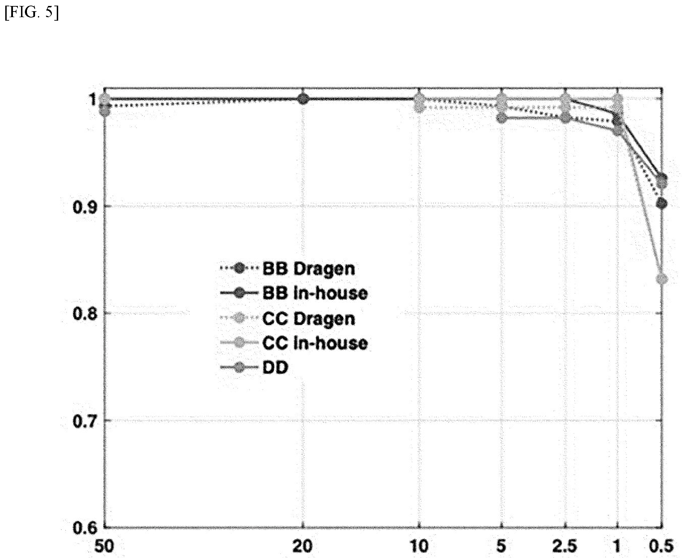

When sensitivity was measured by whether a reference base is detected based on a reference base dilution ratio, results could be obtained showing that 80% or more was detected up to 0.5% AF (allele fraction) under all conditions such as several companies and in-house and DRAGEN analysis, and it could also be seen that differences among companies and analysis methods disappeared (FIG. 5). These results confirmed a bias (reference base bias) by confirming that sensitivity was significantly increased compared to sensitivity (1 to 25%) measured based on an actual variant, and these results suggest that an incorrect conclusion may be drawn if sensitivity is measured based on whether a reference base is detected.

In summary, although the variant detection rate was merely converted into a reference base detection rate for measuring sensitivity, results were obtained indicating that the T-NGS sensitivity of all companies increased, and these results mean that a reference base bias may exist in a bioinformatics analysis process, and suggest a possibility that a VVR error is mostly a simple error repeatedly occurring in a bioinformatics analysis process rather than an actual error. Therefore, removing a VVR error related to a reference base bias occurring in a bioinformatics analysis process and determining a total false positive error rate would be a method of determining an accurate false positive error rate.

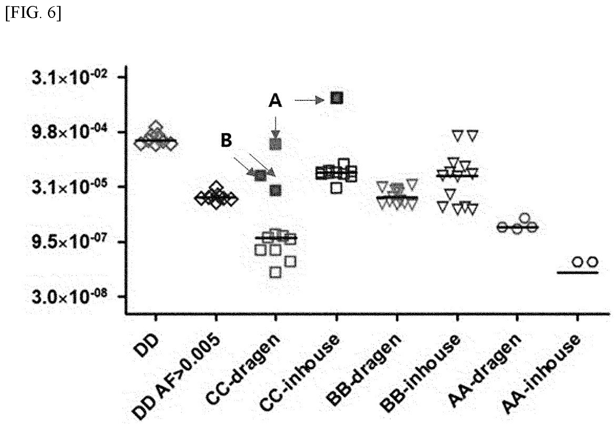

As described above, it was confirmed that reference base bias can cause errors in determining sensitivity and specificity in a bioinformatics analysis process, and based on this, after removing VVR errors in which reference bases are detected in V-V pairs, the total false positive error rate of each T-NGS was calculated, and the results are shown in FIG. 6.

Several other facts could be found in the results of the total false positive error rate, specifically, it could be confirmed that false positive error rates analyzed based on raw data production by one company differed even within one batch and also showed a batch effect in which results may differ between batches.

A box filled with a red color (a box indicated by A in the drawing) in CC-dragen and CC-inhouse of FIG. 6 is a CH10 sample in which control genomic DNA and H. mole DNA sent to company CC at a time different from other samples are mixed at 90:10, and a false positive error rate was 100~1000 times higher only in the CH10 sample compared to samples sent at the same time. This appears to indicate that an error specific to this sample occurred in a production process of raw data. These results mean that some of samples sent in one batch may have an especially high false positive error rate unlike results of other samples, and if a result of such a sample is used in a final analysis, there is a risk that a significant error occurs in a research conclusion.

In addition, the two sample results represented by green-filled boxes (boxes indicated by B in the drawing) in CC-dragen of FIG. 6 are samples independently sent for sensitivity investigation, and are results of samples which produced raw data in a batch different from other samples analyzed in company CC. The results of variant analysis using DRAGEN (CC-dragen false positive error rate in Fig. 6) showed a false positive error rate approximately 100 times higher compared to the results of analyzing other samples. These results imply that a batch effect is shown in a false positive error rate when NGS samples are sent at different times, respectively, and mean that a result of one company does not always produce NGS results of a constant quality. Unlike an error in one sample, this means that a false positive error rate of all samples sent at once may be significantly different from an expected value, and means that it is necessary to examine a batch effect by surely measuring a false positive error rate when sending a sample to be analyzed.

The above results mean that in order to secure reliability of NGS raw data, a reference standard material capable of testing sensitivity and specificity must be sent simultaneously with a clinical sample or a research sample to produce NGS raw data, and NGS results of samples sent to a batch simultaneously can be reliable only when the sensitivity and specificity as a test result of such a standard material meet a certain quality.

### Example 4. Comparative Analysis of Expected VAF and Measured VAF According to Dilution Ratio

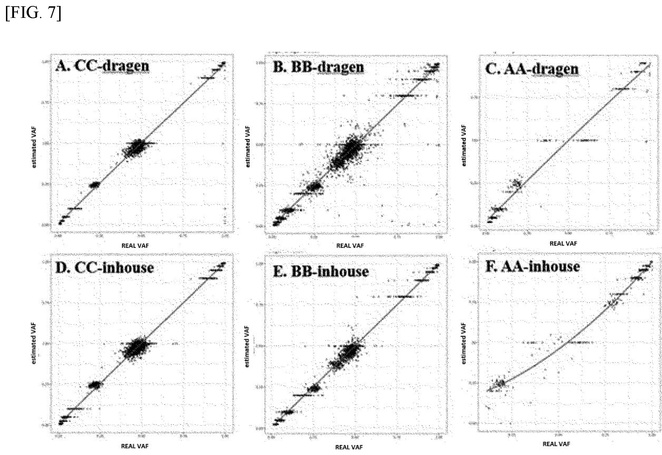

In Ho-N pair and He-N pair alleles used to measure sensitivity, a correlation between an expected VAF according to a dilution ratio and an actual VAF was analyzed, and as shown in FIG. 7, in two of the three companies, the results of calling variants with in-house and DRAGEN showed a linear correlation.

However, in a case of company AA, a correlation was linear when analyzed with DRAGEN, whereas a correlation of a result analyzed by an in-house method did not show a linear relationship as shown in FIG. 7F. Especially, when an expected VAF is low, it is hardly observed in an actual VAF, and values of the actual VAF seem to be higher than expected VAF values. These results mean that a problem occurred in detection of a variant having a low VAF value in the result of detecting variants by a bioinformatics method of company AA, and suggest that improvement of the bioinformatics method of company AA is necessary.

### Example 5. Analysis of Variant Read Count by Chromosome Position

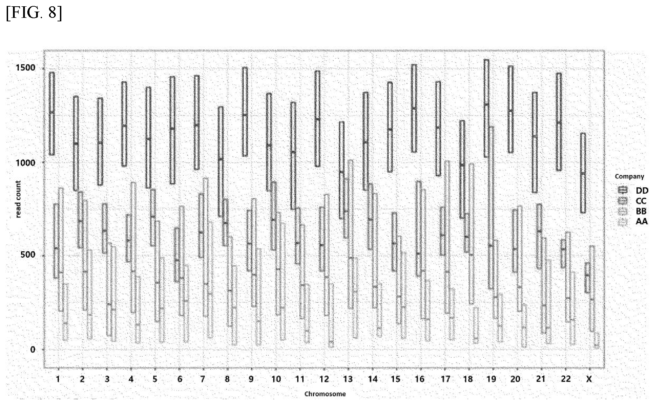

Four NGS service companies were requested to perform analysis at a read depth of about 1000x, but when an actual read count of each variant was shown by chromosome region, a read count of each variant was less than 1000x in NGS results of three companies, and the result of each company also had a lot of differences in a read count by chromosome position (FIG. 8). In a case of company CC, a read count was larger compared to companies AA and BB, and a difference among respective chromosomes was also small. However, in a case of company AA, a total read count was significantly small, and a read count deviation among positions was also large. In a case of company BB, a median read count was smaller compared to company CC, but a read count deviation was much larger. A median read depth of company DD uniquely exceeded 1000x, and a read count by each chromosome was generally constant, but a deviation of a read count was relatively large compared to company CC.

When analyzing by connecting the above results of the read count by chromosome region and the results of sensitivity of each company, although the NGS result of company CC had a larger read count compared to companies AA and BB, a deviation among regions was small, so that it can be said to have a condition in which an FN error for a mutation of a target gene can be small, and these results may be related to relatively high sensitivity of company CC even in the sensitivity results of each company. Therefore, analyzing such a variant read count by chromosome region can be one method of analyzing reliability of an NGS result.

### Example 6. Selection of Chromosome Region in Which Error Rate is Found at High Frequency and Improvement of NGS Analysis Method

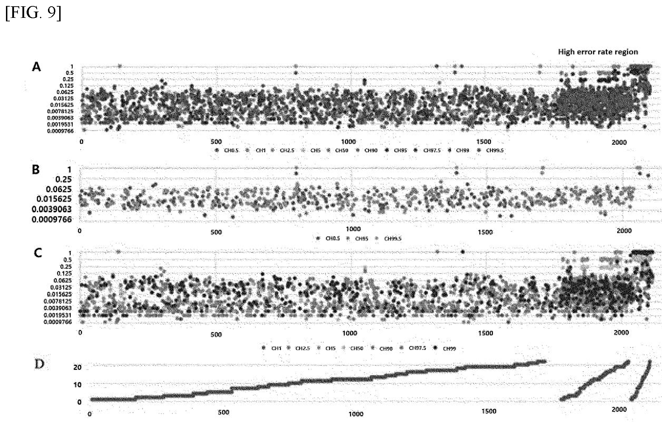

In order to confirm whether false positive errors are concentrated in a specific region by graphically displaying allele fractions (AFs) and positions of false positive bases in which false positive errors come out, a distribution of alleles in which false positive errors appear in an NGS result of company CC was analyzed (FIG. 9).

As a result, it shows an appearance of being evenly distributed in all chromosome regions without appearing in a specific region. A shows false positive errors in total mixture samples; B shows false positive errors in three types of mixture samples such as 99.5:0.5 (CH0.5); 5:95 (CH95), and 0.5:99.5 (CH99.5); C shows false positive errors in the remaining mixture samples excluding the three types of mixture samples; and D shows false positive error alleles divided into three types, wherein the left side shows a distribution of alleles in which three or less errors appeared, the middle shows a chromosome distribution of alleles in which four to six errors appeared, and the right side shows a distribution of alleles in which seven or more errors appeared.

As shown in FIG. 9A, there exist alleles in which errors intensively appear, and there were regions in which four or more false positive errors appear in analysis results of 10 mixture samples. Especially, when seven or more false positive errors appear, it could be seen that a size of an AF of a false positive base is relatively large. Excluding alleles in which such false positive errors appear at a high frequency from a final mutation analysis result can be a method of preventing reporting of false positive errors, and by reporting such regions to a researcher or a patient in advance, errors occurring due to interpretation of research results can be prevented.

In addition, as shown in FIG. 9B, it can be confirmed that in three mixture samples in which control genomic DNA and H. mole DNA are mixed at 99.5:0.5 (CH0.5), 5:95 (CH95), and 0.5:99.5 (CH99.5), unlike other mixture samples, false positive errors of alleles in which false positive errors appear at a high frequency are not high. Considering that a reference standard material was constant in the present study and thus the mixture samples were different only in the mixing ratios of the two samples, the cause of such a batch effect can be said to occur in a production process of NGS raw data. That is, it can be thought to be not an error occurring from a difference in sample DNA or bioinformatics analysis, but an error occurring from a difference in experimental materials used for production of NGS raw data, a slight difference in sample concentrations in an experimental process using the materials, or the use of different PCR instruments. Therefore, it can be said that, if a condition in which occurrence of errors at such an allele region is small is selected in an experimental process of each laboratory, there is a possibility that errors of total NGS will also be reduced. In addition, alleles in which errors especially occur frequently and there is a difference in an error rate can be used as an indicator for reducing errors occurring in a specific NGS kit.

### Example 7. Analysis of Sensitivity and Specificity of Next-Generation Sequencing (NGS) Panel Through Preparation of Formalin fixed Paraffin embedded (FFPE) Cell Block of Mixed Cell Sample

Generally, a process of providing a reference standard material provides a sample prepared in a form of a formalin fixed paraffin embedded (FFPE) tissue using a cancer tissue or a fresh cancer cell, which is used for measurement of sensitivity and specificity of T-NGS detecting a mutation. This is because compared to a case where DNA isolated from a fresh cancer tissue or a fresh cancer cell is used as a reference standard material, when a formalin fixed paraffin embedded (FFPE) cancer tissue or cancer cell is provided as a reference standard material and DNA isolated therefrom is used, there are many false negative errors and false positive errors of measured data.

Here, a fresh cell or a fresh tissue refers to a cell in a living state obtained from culture or a tissue directly isolated from a living body, and may also include a frozen tissue or cell in a state that has not been subjected to chemical treatment. Currently, in order to analyze a mutation in a cancer tissue by an NGS method, an FFPE tissue slide prepared using a tissue containing 50% or more, preferably 75% or more of a cancer tissue is usually used as a reference standard material.

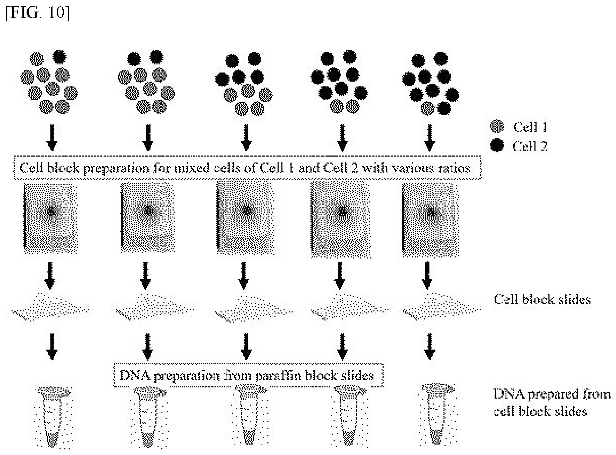

Therefore, in order to provide a composition for validation of NGS panel for utilizing the validation method of the present disclosure in a form of a reference standard material, two types of cells such as an H. mole cell and a control cell were mixed, an FFPE cell block was prepared and then a cell block slide was made to be provided as a reference standard material, or DNA was isolated from a cell block slide to be provided as a reference standard material. That is, when providing a reference standard material, it may be provided in a state of a paraffin embedded tissue slice on a slide, or DNA extracted from a paraffin embedded tissue slice may also be provided (FIG. 10).

In this case, two types of cells may be composed of a homozygote cell, such as an H. mole cell, and a cell having heterozygote alleles, such as a normal blood cell, respectively. Alternatively, both may be composed of H. mole cells having different variants.

When DNA from an FFPE cell block of a mixed cell sample is used, 1) in the case where a mixed cell sample is prepared using a homozygote cell and a control cell, determination of the sensitivity and specificity of NGS is the same as when a sample prepared by mixing DNA is used. However, for NGS analysis of the homozygote cell and the control cell, either fresh cell DNA or FFPE cell block DNA may be used. Since NGS results obtained using DNA from an FFPE cell block exhibit lower sensitivity and specificity, it would be advantageous to use fresh cell DNA. Here, the control cell may be a normal 2n cell or a cancer cell, and, unlike a homozygote cell in which all alleles are composed of homozygote, may be a cell in which alleles are composed of a mixture of heterozygotes and homozygotes.

2) When sensitivity of NGS is determined by preparing a mixed cell sample FFPE cell block using two types of homozygote cells having different variants, heterozygote alleles present in control DNA are absent (He-N pair and Ho-He-N pair are scarcely present), and homozygote alleles are present, so that sensitivity is analyzed using only a Ho-N pair. When specificity of NGS is determined by mixing two homozygote cells, specificity may be determined by distinguishing and analyzing an R-R pair, an R-V pair, and a V-V pair in the same manner as in the analysis of a mixture of homozygote DNA and control DNA.

When two types of homozygote cells having different variants and a control cell are mixed and used, or their DNA is used, an expected VAF may be determined according to a Ho-N pair, a He-N pair, and a Ho-He-N-pair, and sensitivity may be determined in the same method as in analysis of a mixture of a homozygote and control DNA. In addition, specificity may be determined in the same manner as in the analysis of a mixture of homozygote DNA and control DNA by classifying into an R-R pair, an R-V pair, a V-V pair, and the like.

From the above description, those skilled in the art to which the present disclosure pertains will understand that the present disclosure may be embodied in other specific forms without changing the technical spirit or essential characteristics thereof. In this regard, it should be understood that the embodiments described above are illustrative in all respects and not restrictive. The scope of the present disclosure should be construed as including the meaning and scope of the claims to be described later rather than the detailed description above, and all changed or modified forms derived from the equivalent concepts thereof.

## Claims

1. A method for validation of a next-generation sequencing (NGS) panel through analysis of a limit of detection, the method comprising:
(a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting Ho-N pair alleles (Homozygote-Null pair alleles) and/or He-N pair alleles (Heterozygote-Null pair alleles) from the results of the next-generation sequencing (NGS) performed in the (a);
(c) analyzing a diluted variant detection rate, expressed as a percentage of the number of diluted variants found among the Ho-N pair alleles and He-N pair alleles in the DNA mixture sample, based on the selected Ho-N pair alleles and/or He-N pair alleles; and
(d) analyzing a limit of detection.

2. The method of claim 1, wherein the DNA mixture sample in which two or more cell-derived DNAs selected from the group consisting of homozygote DNA, control genomic DNA, and combinations thereof are mixed is a mixture of two or more homozygote DNAs having different variants; a mixture of homozygote DNA and control genomic DNA; or a combination thereof.

3. The method of claim 1, wherein the homozygote DNA is DNA isolated from a hydatidiform mole cell line or a parthenogenetic cell line.

4. The method of claim 1, wherein the control genomic DNA is homozygote DNA; heterozygote DNA; or genomic DNA isolated from blood cells or tissue cells of a normal subject, cultured blood cells, tissue cells or cancer cells, or blood cells or cancer tissue cells of a patient.

5. The method of claim 1, wherein the homozygote DNA or the control genomic DNA is isolated from fresh cells, fresh tissues, or formalin fixed paraffin embedded (FFPE) cellular tissues.

6. A method for validation of a next-generation sequencing (NGS) panel through analysis of a false positive error rate, the method comprising:
(a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting false positive variants from the results of the next-generation sequencing (NGS) performed in the (a); and
(c) analyzing a false positive error rate in the DNA mixture sample based on the selected false positive variants.

7. The method of claim 6, wherein the DNA mixture sample in which two or more cell-derived DNAs selected from the group consisting of homozygote DNA, control genomic DNA, and combinations thereof are mixed is a mixture of two or more homozygote DNAs having different variants; a mixture of homozygote DNA and control genomic DNA; or a combination thereof.

8. The method of claim 6, wherein the false positive variant is a case in which a variant is shown to exist in the result of analyzing a DNA mixture sample by NGS among null alleles in which no variant is found in any of two or more cell-derived DNAs selected from the group consisting of homozygote DNA, control genomic DNA, and combinations thereof.

9. The method of claim 6, wherein the false positive variant is a case in which a variant other than the variants of the V-V pair or a reference base is found in the result of analyzing a DNA mixture sample by NGS among alleles (V-V pairs) in which no reference base is found and only variants are all found in two or more cell-derived DNAs selected from the group consisting of homozygote DNA, control genomic DNA, and combinations thereof.

10. The method of claim 6, wherein the false positive variant is a case in which a new variant other than the bases constituting an R-V pair is found in the result of analyzing a DNA mixture sample by NGS among alleles (R-V pair alleles) in which a reference base and a variant are mixed in two or more cell-derived DNAs selected from the group consisting of homozygote DNA, control genomic DNA, and combinations thereof.

11. The method of claim 9, wherein the (c) further comprises removing a VVR false positive error result in which a reference base is found in a V-V pair.

12. The method of claim 6, wherein
the (c) further comprises excluding error results of alleles in which false positive errors appear at a high frequency.

13. The method of claim 6, wherein the (c) further comprises examining a batch effect.

14. A method for validation of a next-generation sequencing (NGS) panel, the method comprising:
(a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting Ho-N pair alleles (Homozygote-Null pair alleles) and/or He-N pair alleles (Heterozygote-Null pair alleles) from the results of the next-generation sequencing (NGS) performed in the (a);
(c) analyzing a diluted variant detection rate, expressed as a percentage of the number of diluted variants found among the Ho-N pair alleles and He-N pair alleles in the DNA mixture sample, based on the selected Ho-N pair alleles and/or He-N pair alleles; and
(d) comparing and analyzing an expected VAF and an actual VAF according to the dilution ratio.

15. The method of claim 14, wherein the DNA mixture sample in which two or more cell-derived DNAs selected from the group consisting of homozygote DNA, control genomic DNA, and combinations thereof are mixed is a mixture of two or more homozygote DNAs having different variants; a mixture of homozygote DNA and control genomic DNA; or a combination thereof.

16. The method of claim 14, wherein the homozygote DNA or the control genomic DNA is isolated from fresh cells, fresh tissues, or formalin fixed paraffin embedded (FFPE) cellular tissues.

17. A method for validation of a next-generation sequencing (NGS) panel, the method comprising:
(a) performing next-generation sequencing (NGS) using a next-generation sequencing (NGS) panel on a DNA mixture sample in which two or more cell-derived DNAs selected from the group consisting of homozygote DNA, control genomic DNA, and combinations thereof are mixed;
(b) selecting Ho-N pair alleles (Homozygote-Null pair alleles) and/or He-N pair alleles (Heterozygote-Null pair alleles) from the results of the next-generation sequencing (NGS) performed in the (a);
(c) analyzing a diluted variant detection rate, expressed as a percentage of the number of diluted variants found among the Ho-N pair alleles and He-N pair alleles in the DNA mixture sample, based on the selected Ho-N pair alleles and/or He-N pair alleles; and
(d) analyzing deviations in variant read counts for respective chromosomal regions used for detection of diluted variants in (c) above.

18. The method of claim 17, wherein the DNA mixture sample in which two or more cell-derived DNAs selected from the group consisting of homozygote DNA, control genomic DNA, and combinations thereof are mixed is a mixture of two or more homozygote DNAs having different variants; a mixture of homozygote DNA and control genomic DNA; or a combination thereof.

19. The method of claim 17, wherein the homozygote DNA or the control genomic DNA is isolated from fresh cells, fresh tissues, or formalin fixed paraffin embedded (FFPE) cellular tissues.

20. A composition for validation of a next-generation sequencing (NGS) panel, comprising a DNA mixture in which two or more cell-derived DNAs selected from the group consisting of homozygote DNA, control genomic DNA, and combinations thereof are mixed.

21. The composition of claim 20, wherein the DNA mixture in which two or more cell-derived DNAs selected from the group consisting of homozygote DNA, control genomic DNA, and combinations thereof are mixed is a mixture of two or more homozygote DNAs having different variants; a mixture of homozygote DNA and control genomic DNA; or a combination thereof.

22. The composition of claim 20, wherein the homozygote DNA is DNA isolated from a hydatidiform mole cell line or a parthenogenetic cell line.

23. The composition of claim 20, wherein the control genomic DNA is homozygote DNA; heterozygote DNA; or genomic DNA isolated from blood cells or tissue cells of a normal subject, cultured blood cells, tissue cells or cancer cells, or blood cells or cancer tissue cells of a patient.

24. A composition for validation of a next-generation sequencing (NGS) panel, comprising a mixture in which two or more cells selected from the group consisting of homozygote cells, control cells, and combinations thereof are mixed.

25. The composition of claim 24, wherein the mixture in which two or more cells selected from homozygote cells, control cells, or combinations thereof are mixed is a mixture of two or more homozygote cells having different variants; a mixture of two or more homozygote cells having different variants and control cells; a mixture of homozygote cells and control cells; or a combination thereof.

26. The composition of claim 24, wherein the homozygote cells or the control cells are fresh cells or formalin fixed paraffin embedded (FFPE) cells.

27. The composition of claim 24, wherein the composition comprises, in the form of a formalin fixed paraffin embedded (FFPE) cell block, a mixture in which two or more cells selected from homozygote cells, control cells, or combinations thereof are mixed.

28. The composition of claim 24, wherein the homozygote cell is a hydatidiform mole cell or a parthenogenetic cell.

29. The composition of claim 24, wherein the control cell is a homozygote cell; a heterozygote cell; a blood cell or tissue cell of a normal subject; a cultured blood cell, tissue cell, or cancer cell; or a blood cell or cancer tissue cell of a patient.
